# EUROPEAN PATENT APPLICATION

(11) **EP 3 107 080 A1**
(43) Date of publication of application: **21.12.2016**
(21) Application number: 15748495.7
(22) Date of filing: 06.02.2015
(51) Int. Cl.: G09B 23/28, A01N 1/02, G09B 9/00

(54) **PSEUDO-HUMAN BODY DEVICE**

(30) Priority: 12.02.2014 JP 2014024910
(71) Applicant: Olympus Corporation, Tokyo 192-8507 (JP)
(72) Inventor: TSUKIYAMA, Shusaku, Tokyo 192-8507 (JP); NAKAMURA, Koki, Tokyo 192-8507 (JP); SAKAO, Satomi, Tokyo 192-8507 (JP); SUZUKI, Keiko, Tokyo 192-8507 (JP); TANIGAMI, Yasuo, Tokyo 192-8507 (JP); SUZUKI, Takayuki, Tokyo 192-8507 (JP)
(74) Representative: Schicker, Silvia
(86) International application number: PCT/JP2015/053349
(87) International publication number: WO 2015/122363

(57) **Abstract**

A pseudo human body device includes, an animal-derived organ having a living tissue and a blood vessel extending from the living tissue, a blood collecting unit which collects blood to be sent to the blood vessel, a tube which connects the blood vessel to the blood collecting unit, a pump unit which is provided along the tube and which sends the blood to the blood vessel from the blood collecting unit, and a housing unit which houses the organ and which sends steam of a temperature higher than a room temperature to the organ to humidify the organ and bring the organ to a temperature of 20°C to 50°C.

## Description

The present invention relates to a pseudo human body device for use in the evaluation of therapeutic equipment such as a treatment device or an electric scalpel.

### Background Art

Jpn. Pat. Appln. KOKAI Publication No. 2003-206201 (Patent Literature 1) discloses an artificial organ system including a liver housing device which holds, for example, a liver for transplant in a functional state.

### Citation List

### Patent Literature

Patent Literature 1: Jpn. Pat. Appln. KOKAI Publication No. 2003-206201

### Summary of Invention

### Technical Problem

A patient may bleed during a treatment such as surgery or a treatment using an endoscope, and various kinds of therapeutic equipment such as a treatment device or an electric scalpel may be used to stop the bleeding. Various kinds of therapeutic equipment such as a treatment device or an electric scalpel are also used in the surgery or the procedure using the endoscope. To improve the performance of the above equipment, an experimental system capable of evaluating the performance of the equipment is needed. However, in the present situation, there has not yet been fully established any experimental system capable of evaluating the performance of the above equipment.

For purposes other than the performance evaluation of the equipment, the present experimental system is considered to be effectively utilized in the situation of training performed by doctors to learn surgery and treatment techniques using an endoscope and in the situation of demonstrations that are given so that the doctors who are the users may fully understand the performance of developed equipment. There have been demands for development of an experimental system which can be put into practical use in a simple and easy manner for a wide range of purposes.

Here, the following equipment and action mechanisms thereof to be evaluated are conceivable:
(1) an electric scalpel which is intended to cut open a living tissue and which uses energy such as a high-frequency electric current or heat to coagulate and cut open a tissue at the same time and thus suppress bleeding caused by the cutting;
(2) a hemostatic treatment device which is intended to stop bleeding from a living tissue and which stops the bleeding by coagulating the tissue or blood by energy such as a high-frequency electric current or heat;
(3) a hemostatic treatment device which is intended to stop bleeding from a living tissue and which stops the bleeding by mechanically compressing and sealing a bleeding tissue or a blood vessel end with a treatment portion shaped like a forceps or with a retaining tool such as a clip or a ligature tool; and
(4) therapeutic equipment which is intended to coagulate/cauterize and thereby treat a living tissue and which disposes an output terminal inside a target tissue and outputs energy such as a high-frequency electric current or heat to form a region for the coagulation/cautery in the living tissue and thereby treat the living tissue.

An object of the present invention is to provide a pseudo human body device capable of stably evaluating the performance of therapeutic equipment such as a treatment device or an electric scalpel.

A pseudo human body device includes, an animal-derived organ having a living tissue and a blood vessel extending from the living tissue, a blood collecting unit which collects blood to be sent to the blood vessel, a tube which connects the blood vessel to the blood collecting unit, a pump unit which is provided along the tube and which sends the blood to the blood vessel from the blood collecting unit, and a housing unit which houses the organ and which sends steam of a temperature higher than a room temperature to the organ to humidify the organ and bring the organ to a temperature of 20°C to 50°C.

### Advantageous Effects of Invention

According to the configuration described above, it is possible to provide a pseudo human body device capable of stably evaluating the performance of a treatment device. Brief Description of Drawings
FIG. 1 is a schematic diagram showing the overall configuration of a pseudo human body device according to a first embodiment;
FIG. 2 is a schematic diagram showing the overall configuration of the pseudo human body device according to a second embodiment;
FIG. 3 is a schematic diagram showing the overall configuration of the pseudo human body device according to a third embodiment;
FIG. 4 is a schematic diagram showing an artery side end of a blood vessel and a first plug of the pseudo human body device shown in FIG. 3;
FIG. 5 is a sectional view taken along the longitudinal direction of the first plug shown in FIG. 4;
FIG. 6 is a schematic diagram showing a second plug and a vein side end of a blood vessel according to a modification of the pseudo human body device in the third embodiment;
FIG. 7 is a sectional view taken along the longitudinal direction of the second plug shown in FIG. 6;
FIG. 8 is a sectional view showing a modification of the second plug shown in FIG. 7;
FIG. 9 is a schematic diagram showing the overall configuration of the pseudo human body device according to a fourth embodiment;
FIG. 10 is a schematic diagram showing the overall configuration of the pseudo human body device according to a fifth embodiment;
FIG. 11 is a schematic diagram showing stimulus supply means of the pseudo human body device according to a sixth embodiment;
FIG. 12 is a schematic diagram showing a modification of the stimulus supply means shown in FIG. 11;
FIG. 13 is a schematic diagram showing a vibration applying unit of the pseudo human body device according to a seventh embodiment;
FIG. 14 is a schematic diagram showing a modification of the vibration applying unit shown in FIG. 13;
FIG. 15 is a schematic diagram showing the overall configuration of the pseudo human body device according to an eighth embodiment; and
FIG. 16 is a schematic diagram showing the overall configuration of the pseudo human body device according to a ninth embodiment.

### Description of Embodiments

### [First Embodiment]

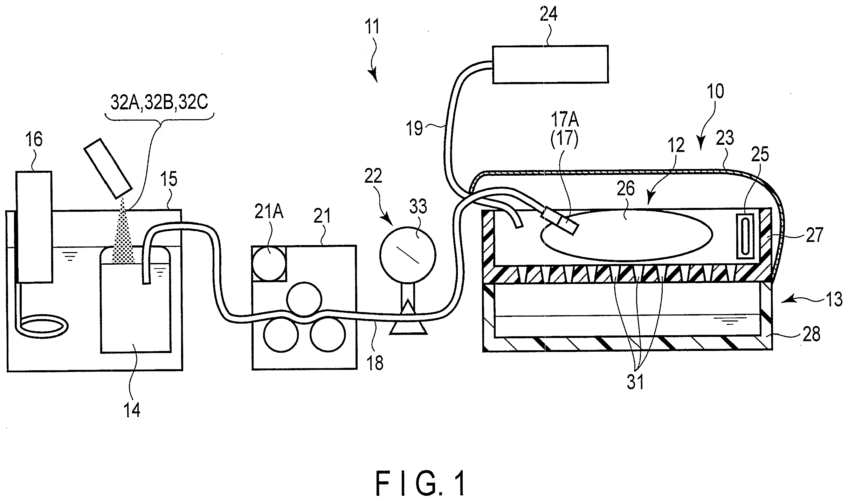

A pseudo human body device according to the embodiment can be used in the evaluation of equipment to perform a treatment; for example, cut open a living tissue and stop its bleeding (coagulate). The overall configuration of a pseudo human body device 11 is shown in FIG. 1.

As shown in FIG. 1, the pseudo human body device 11 has an animal-derived organ 12, a mounting unit 13 to mount the organ 12, a blood bottle 14 which stores blood to be sent to the organ 12, a constant temperature bath 15 in which the blood bottle 14 is put, a heater 16 (hot water heater) which heats water in the constant temperature bath 15 at a constant temperature, a tube 18 which connects the blood bottle 14 to an artery side end 17A of a blood vessel 17 of the organ 12, a pump unit 21 and a pressure sensor 22 which intervene in the tube 18 of the organ 12, a cover 23 which surrounds the mounting unit 13 to substantially tightly close the organ 12, a high-temperature steam generator 24 which sends steam inside the cover 23, and a thermometer/hygrometer 25 attached to the mounting unit 13 inside the cover 23. A housing unit 10 which can house the organ 12 in a substantially tightly closed state is constituted by the mounting unit 13, the cover 23, and the high-temperature steam generator 24.

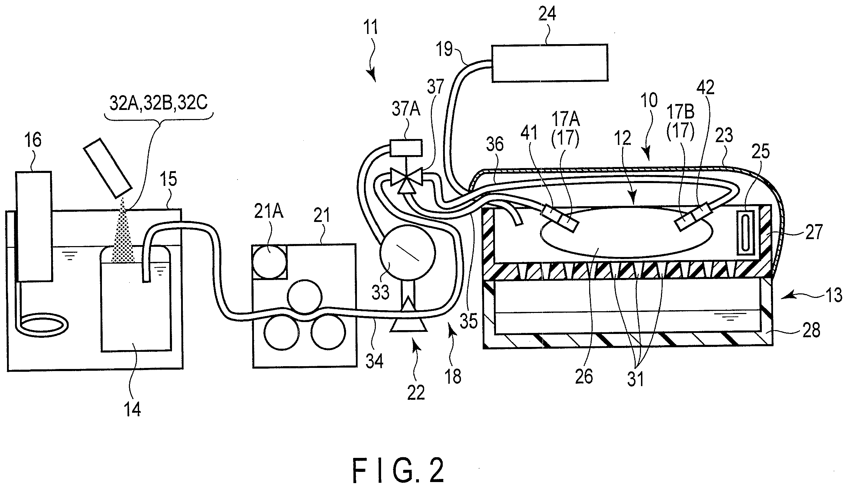

The organ 12 is obtained from a living body such as a domestic animal for meat such as a pig or a cow, and can be purchased from meat traders. The organ 12 includes a living tissue 26, and the blood vessel 17 extending from the living tissue 26 and which supplies blood to the living tissue 26. The blood vessel 17 includes the artery side end 17A and a vein side end 17B (see FIG. 2 and others). The living tissue 26 used in the present embodiment includes an esophagus, a stomach, a small intestine, a large intestine, a duodenum, a liver, a mesentery, a heart, a spleen, a kidney, a lung, an eyeball, and other transplantable organs, as well as a muscular tissue such as a skeletal muscle, a dermal tissue, a blood vessel, and nervous tissues of, for example, a brain.

The mounting unit 13 has a box-shaped first tray 27, and a box-shaped second tray 28 laid under the first tray 27. The first tray 27 has through-holes 31 in the bottom surface, and can drop down the blood which has flowed out of the organ 12 through the through-holes 31. The first tray 27 is made of, for example, a transparent resin. The second tray 28 can collect the blood dropped from the through-holes 31. The second tray 28 is made of, for example, a resin.

A transparent bottle is used as the blood bottle 14. The blood bottle 14 is an example of a blood collecting unit. The blood comprises generally available animal-derived blood (e.g. pig blood). At least one of an anticoagulant 32A, a thrombolytic agent 32B, a vasodilator 32C is preferably mixed in the blood in advance. Ethylene diamine tetraacetic acid (EDTA), heparin, sodium citrate, sodium fluoride, or an acid citrate dextrose (ACD) solution can be used as the anticoagulant 32A. The abovementioned anticoagulant that does not hinder the original coagulation function of the blood attributed to heating is preferably used particularly in the evaluation of the equipment which uses energy such as a high-frequency electric current or heat to coagulate a living tissue or blood and thereby stop bleeding.

The thrombolytic agent 32B can dissolve a thrombus which hinders the perfusion of blood. Alteplase, monteplase, or pamiteplase which is a tissue-type plasminogen activator (t-PA) can be used as the thrombolytic agent 32B, so that the thrombus can be dissolved by plasmin which is formed by the activation of plasminogen. A plasmin preparation can also be used as the thrombolytic agent 32B, and the thrombus may be directly dissolved by the plasmin preparation.

A calcium antagonist, a nitrous acid drug, a renin inhibitor, an angiotensin converting enzyme inhibitor, an angiotensin receptor antagonist, an alpha-blocking drug, or a beta stimulator can be used as the vasodilator 32C.

Hot water is collected within the constant temperature bath 15. The heater 16 is a general looped heater, and can apply heat to the hot water in the constant temperature bath 15. The tube 18 is formed into a tubular shape by, for example, a transparent resin so that the flow of the blood running therethrough can be visually recognized. The tube 18 includes, at its end on the side of the organ 12, a sonde for insertion into a blood vessel. The constant temperature bath 15 and the heater 16 are examples of a temperature adjusting unit which applies heat to the blood bottle 14 and warms the blood bottle 14 to a predetermined temperature and keeps the blood bottle 14 warm.

The pump unit 21 comprises a general roller pump (tube pump), and includes a flow volume adjusting unit 21A for adjusting the flow volume of a fluid (blood) flowing through the tube 18. The flow volume adjusting unit 21A can adjust the flow volume or supply pressure of the fluid (blood) flowing through the tube 18 by the rotation number of a pump rotor.

A generally available pressure sensor can be used as the pressure sensor 22. The pressure sensor 22 is an example of a sensor unit. The pressure sensor 22 can convert the pressure of the fluid (blood) flowing through the tube 18 into an electric signal by a built-in pressure transducer. The electric signal thus acquired is output to an attached data logger 33 (an instrument for measuring and saving sensor signals), and displayed as a numerical value on a display unit of the data logger 33 or stored in a storage device in the data logger 33.

The pressure sensor 22 is provided, for example, in the vicinity of the end of the tube 18 on the side of the organ 12. Here, providing in the vicinity of the end of the tube 18 on the side of the organ 12 means providing the pressure sensor 22 in the part of the tube 18 on the side of the organ 12 within a range of length which is about one third of the entire length of the tube 18 (preferably, 10% to 20% of the entire length of the tube 18). The pressure sensor 22 is provided in the vicinity of the end of the tube 18 on the side of the organ 12 so that the pressure of the blood on the inside of the living tissue 26 of the organ 12 can be accurately sensed.

The cover 23 comprises a transparent bag made of, for example, a synthetic resin. A general steam generator and steam cleaner can be used as the high-temperature steam generator 24. The high-temperature steam generator 24 includes a second tube 19 to guide high-temperature steam into the cover 23. The high-temperature steam generator 24 is an example of a heater/humidifier unit. Although a general thermometer/hygrometer can be used, a function of connecting and disconnecting the supply of the high-temperature steam may be added, for example, by providing an upper limit and a lower limit of a set temperature.

Now, a manufacturing method of the pseudo human body device 11 is described. First, the organ 12 purchased from a meat trader is immersed in a water bath (constant temperature bath) kept warm at 37°C, and heated for about one hour. In this way, adipose tissues in the organ 12 are softened to eliminate the compression and crushing of the blood vessel.

In a similar manner, animal blood that has been purchased is put in the blood bottle 14, and the blood bottle 14 is immersed in the water bath (constant temperature bath) kept warm at 37°C. The blood is warmed for about one hour while being stirred by use of, for example, a stirring bar put in the blood bottle 14. In this way, precipitation of the protein constituent having a high specific gravity on the lower side is prevented so that the blood has a uniform viscosity and so that the blood pressure and the flow volume may not change when the blood is perfused.

The high-temperature steam (e.g. steam at 100°C) generated in the high-temperature steam generator 24 is supplied to the inside of the cover 23 to keep an environment inside the cover 23 at a temperature of 20 to 50°C and at a humidity of 50% or more. In this state, the organ 12 is put on the first tray 27 of the mounting unit 13 inside the cover 23, and the organ 12 is kept warm at 20 to 50°C. In this way, a drop in the temperature of the organ 12 is prevented to prevent the adipose tissues from becoming stiff. Moreover, deterioration of the organ 12 caused by drying is prevented by adequate humidification.

The tube 18 (sonde) is then inserted into the blood vessel 17 (artery) extending from the living tissue 26 (e.g. stomach). The tube 18 is preferably fixed from the outside of the blood vessel 17 by being ligated in at least two or more places with, for example, a strong suture thread.

In this state, the tube 18 is connected to a bottle in which a physiological saline previously warmed at 37°C in the water bath is put as in the blood bottle 14, and the physiological saline is perfused (perfusion pressure: 10 to 760 mmHg, depending on the individual difference) in the organ 12 for, for example, 5 to 15 minutes. In this instance, when the rotation number of the pump unit 21 is set at, for example, 6 rpm, the total perfusion volume of the physiological saline during this period is, for example, about 100 ml to 1 L. In this way, the blood and waste product remaining in the blood vessel are expelled to the outside by the perfusion of the physiological saline having a low pipe resistance, and clogging of the blood vessel is prevented when blood in the next process is perfused. If the physiological saline is perfused for 5 to 15 minutes, almost no waste product is contained in the physiological saline discharged from the vein-side blood vessel 17. If the physiological saline is colored with a pigment, the blood vessel, which is supplied colored saline by the artery side end 17A of the blood vessel 17 to which the tube 18 is connected, is only colored with the pigment, so that which region of the living tissue 26 included in the organ 12 undergoes perfusion can be known before a blood perfusion process. The use of a blue or green pigment is preferred because the difference between the pigment and the original color tone of the living tissue is easily identified.

The tube 18 is then connected to the blood bottle 14, and the blood is perfused in the organ 12, for example, for 5 to 15 minutes (perfusion pressure: 10 to 760 mmHg). The rotation number of the pump unit 21 is 6 rpm, and the total perfusion volume of the blood during this period is, for example, about 100 ml to 1 L. This perfusion of the blood for 5 to 15 minutes allows the blood to be spread over the range of the living tissue 26 supplied by the artery side end 17A of the blood vessel 17. Thus, the cutting performance of therapeutic equipment such as a treatment device or an electric scalpel and bleeding stopping performance (attributed to the coagulation function of the living tissue) can be stably evaluated later. The blood leaking from the organ 12 during the evaluation of the equipment is collected in the second tray 28 from the first tray 27 through the through-holes 31.

The supply pressure of the blood supplied to the organ 12 is changed in consideration of the individual difference of a human body to be reproduced and conditions produced by the state of a disease. Higher pressure of the blood can be set when a high blood pressure state is to be reproduced, whereas lower pressure of the blood can be set when a low blood pressure state is to be reproduced. It is also possible to reproduce a condition of a patient having blood that is difficult to stop by mixing a predetermined drug in the blood.

According to the first embodiment, the pseudo human body device 11 comprises the organ 12 of a domestic animal for meat having the living tissue 26 and the blood vessel 17 extending from the living tissue 26, the blood collecting unit which collects blood to be sent to the blood vessel 17, the tube 18 which connects the blood vessel 17 to the blood collecting unit, the pump unit 21 which is provided along the tube 18 and which sends the blood to the blood vessel 17 from the blood collecting unit, and the housing unit 10 which houses the organ 12 and which sends steam of a temperature higher than the room temperature into the cover 23 to humidify the organ 12 and bring the organ 12 to a temperature of 20°C to 50°C.

According to this configuration, the living tissue 26 and the organ 12 in which the blood circulates can be reproduced by the simple device and configuration. Thus, it is possible to manufacture the pseudo human body device 11 which minimizes the influence of an animal individual difference as compared to an experimental system manufactured by use of a living animal or the like, and stably manufacture a sufficient number of pseudo human body devices 11 necessary for the evaluation of equipment. It is also possible to easily reproduce the pseudo human body device 11 in which mixing a drug into the blood is easy and which assumes various types of patients (e.g. a patient who always takes a drug that suppresses clogging of the blood vessel and who has difficulty in stopping bleeding due to the drug). According to the configuration described above, deterioration of the organ 12 in a short time can be prevented by the prevention of the drying of the organ 12.

The pseudo human body device 11 comprises the temperature adjusting unit which applies heat to the blood collecting unit to keep the temperature of the blood at a set temperature equal to or more than the room temperature. According to this configuration, there is no fear that the organ 12 humidified by the heater/humidifier unit may be again cooled by the blood as compared to the case in which the blood that is not warmed is perfused. It is therefore possible to stably run the pseudo human body device 11.

At least one of an anticoagulant, a thrombolytic agent, a vasodilator is mixed in the blood. According to this configuration, the anticoagulant can prevent the blood from coagulating before the blood is supplied to the organ. A thrombus in the organ 12 can be removed by the thrombolytic agent, and the blood vessel 17 can be dilated by the vasodilator, so that the perfusion efficiency of the blood can be improved. Thus, the flow of the blood can be improved even if low supply pressure of the blood is set. Therefore, when higher supply pressure is set, larger-scale bleeding can be reproduced in the pseudo human body device 11.

A blood flow may be reproduced in the organ 12 by perfusing a liquid other than the blood after the perfusion of the physiological saline. For example, it is possible to use a physiological saline colored with a pigment so that bleeding from the living tissue 26 is easily recognized. In this case, as compared to the case in which the blood is perfused, there is no influence of the precipitation of protein and impurities mixed in the blood, so that a bleeding phenomenon can be more stably and more reproducibly formed.

However, the original coagulation function of the blood can not be reproduced, so that an application to a condition that does not require a coagulation function attributed to heat is preferable if an organ to be applied, a living tissue, and the functions of an organ to be evaluated are taken into consideration.

### [Second Embodiment]

The pseudo human body device 11 according to the second embodiment is described with reference to FIG. 2. The pseudo human body device 11 according to the second embodiment is different from that according to the first embodiment in the configuration of the tube 18 and in that a first plug 41 and a second plug 42 are provided. However, the pseudo human body device 11 according to the second embodiment is the same as that according to the first embodiment in other respects. Therefore, the differences between the first embodiment and the second embodiment are mainly described, and the same parts are neither shown in the drawings nor described.

The tube 18 has a body 34 (a part connected to the blood collecting unit) connected to the blood bottle 14, a first branch 35 and a second branch 36 extending from the body 34, and a switching valve (three-way valve) 37 intervening between the body 34, the first branch 35, and the second branch 36. The pseudo human body device 11 comprises the circular cylindrical first plug 41 intervening between the artery side end 17A of the blood vessel 17 and the first branch 35, and the circular cylindrical second plug 42 intervening between the vein side end 17B of the blood vessel 17 and the second branch 36.

The first plug 41 is formed, for example, into a circular cylindrical shape by a synthetic resin. The second plug 42 is formed, for example, into a circular cylindrical shape by a synthetic resin material.

The first branch 35 and the second branch 36 have a tubular shape similar to that of the body 34. The first branch 35 is connected to the artery side end 17A of the blood vessel 17. The second branch 36 is connected to the vein side end 17B of the blood vessel 17. The switching valve 37 comprises what is known as an electromagnetic valve, and can circulate the blood to one of the first branch 35 and the second branch 36.

The switching valve 37 has a control unit 37A, and the control unit 37A can read the pressure sensed by the pressure sensor 22 (sensor unit). The control unit 37A normally controls the switching valve 37 so that the blood flows to the first branch 35. When the pressure sensed by the pressure sensor 22 is more than predetermined pressure (e.g. twice the originally scheduled pressure in the normal state), the control unit 37A switches the switching valve 37 so that the blood flows to the second branch 36. The predetermined pressure is not exclusively twice the originally scheduled pressure in the normal state, and can be suitably changed in accordance with the states of the organ 12 and the living tissue 26 and the conditions of a human body to be reproduced.

Now, a blood perfusion method using the pseudo human body device 11 according to the present embodiment is described. In a manner similar to the manufacturing method of the pseudo human body device 11 described in the first embodiment, the organ 12 is humidified in the water bath at 37°C, and the blood bottle 14 is humidified in the water bath at 37°C. High-temperature steam (100°C) generated in the high-temperature steam generator 24 is supplied to the inside of the cover 23 to keep an environment inside the cover 23 at a temperature of 20 to 50°C and at a humidity of 50% or more. The first branch 35 (the first plug 41) is then inserted into the blood vessel 17 (the artery side end 17A) extending from the living tissue 26, and the second branch 36 (the second plug 42) is inserted into the blood vessel 17 (the vein side end 17B). The tube 18 is fixed and ligated with, for example, a thread in at least two or more places outside the blood vessel 17. Further, a physiological saline is perfused in the organ 12, for example, for 5 to 15 minutes (perfusion pressure: 10 to 760 mmHg) before the perfusion of the blood, and then the blood is perfused in the organ 12, for example, for 5 to 15 minutes (perfusion pressure: 10 to 760 mmHg). The equipment is then evaluated after the perfusion of the blood.

In this perfusion of the blood, the switching valve 37 is open to the first branch 35 at the beginning, and supplies the blood supplied from the blood bottle 14 to the first branch 35. When the pressure sensed by the pressure sensor 22 is more than twice the originally scheduled blood pressure, the control unit 37A of the switching valve 37 switches the switching valve (electromagnetic valve) 37 so that the blood flows to the second branch 36. Thus, the blood flows back from the vein to the artery in the blood vessel 17. As a result of this backflow of the blood, a thrombus, for example, in the blood vessel 17 comes off the inner wall of the blood vessel so that the blood vessel 17 is unclogged. In the present embodiment, after the backflow of the blood from the vein to the artery for several seconds to several ten seconds, the control unit 37A switches the switching valve 37 so that the blood flows to the first branch 35. In the meantime, a user who evaluates the equipment can evaluate the equipment in both the normal state in which the blood flows from the artery to the vein and the backflow state in which the blood flows from the vein to the artery.

According to the second embodiment, the tube 18 has the part connected to the blood collecting unit, the tubular first branch 35 extending from the above part and connected to the artery side end 17A of the blood vessel 17, the tubular second branch 36 extending from the above part and connected to the vein side end 17B of the blood vessel 17, and the switching valve 37 which intervenes between the above part, the first branch 35, and the second branch 36 and which can circulate the blood to one of the first branch 35 and the second branch 36.

According to this configuration, the blood can be suitably circulated to the blood vessel of the organ 12 in the forward direction and the backflow direction. Thus, when the blood does not flow through the blood vessel 17 well because of a thrombus, the blood is circulated in the backflow direction so that the thrombus can be efficiently removed.

The pseudo human body device 11 comprises the sensor unit which is provided along the tube 18 and which senses the pressure of the blood. The switching valve 37 circulates the blood to the first branch 35 in the normal state, and circulates the blood to the second branch 36 when the pressure of the blood sensed by the sensor unit is more than predetermined set pressure which is abnormally higher than the pressure of the blood in the normal state. According to this configuration, an abnormal increase of the pressure of the blood can be prevented when the blood vessel 17 is clogged. It is therefore possible to prevent an extreme increase of the pressure of the blood that leads to the breakage of the blood vessel 17.

### [Third Embodiment]

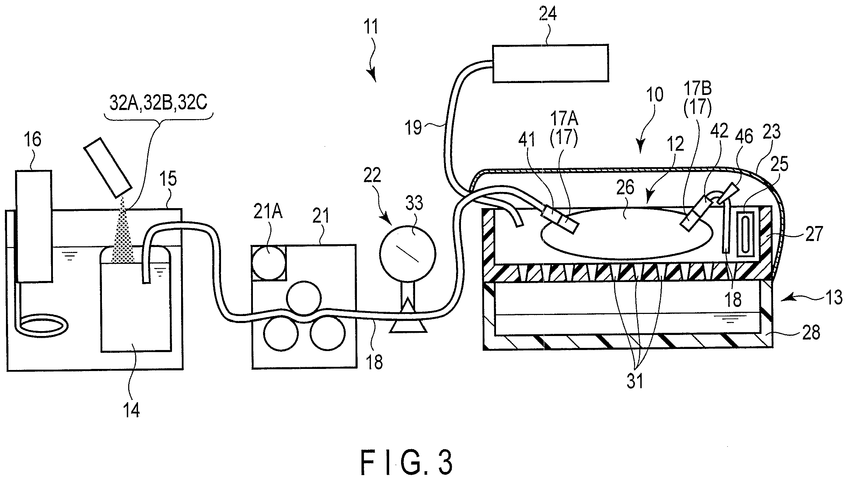

The pseudo human body device according to the third embodiment is described with reference to FIG. 3 to FIG. 5. The pseudo human body device 11 according to the third embodiment is different from that according to the first embodiment in that, a first plug 41 and a clamp member 46 are provided. The first plug 41 is connected to the artery side end 17A. The clamp member 46 is provided to produce outflow resistance by externally closing or narrowing the vein side end 17B or a lumen of the tube 18 connected to the vein side end. However, the pseudo human body device 11 according to the third embodiment is the same as that according to the first embodiment in other respects. Therefore, the differences between the first embodiment and the third embodiment are mainly described, and the same parts are neither shown in the drawings nor described.

The pseudo human body device 11 comprises the circular cylindrical first plug 41. The first plug 41 intervenes between the artery side end 17A of the blood vessel 17 and the tube 18.

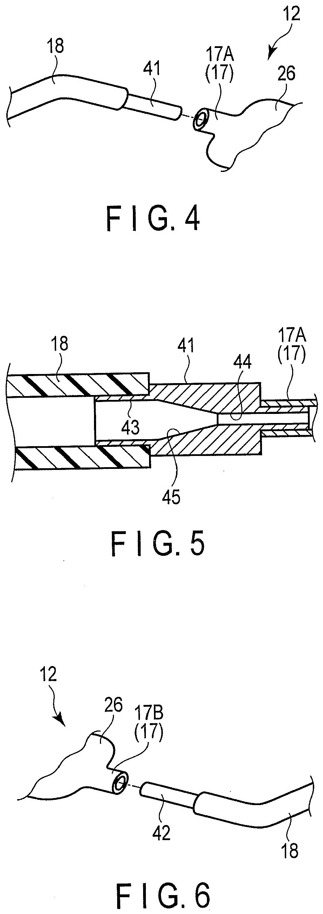

As shown in FIG. 4 and FIG. 5, the first plug 41 has a first circular cylindrical portion 43, a first minor diameter portion 44 projecting inward from the first circular cylindrical portion 43, and an inclined portion 45 that links the first circular cylindrical portion 43 and the first minor diameter portion 44. The first circular cylindrical portion 43 has an inside diameter less than or equal to the inside diameter of the tube 18. The first minor diameter portion 44 constitutes an inside diameter smaller than the inside diameter of the first circular cylindrical portion 43. The inclined portion 45 is funnel-shaped to smoothly connect the first circular cylindrical portion 43 and the first minor diameter portion 44.

The clamp member 46 has the shape of what is known as a medical forceps, a clothespin, or a clip. The clamp member 46 is not limited to the shape of the forceps, the clothespin, or the clip, and may have any shape that can hold the blood vessel 17 or the tube 18 in between. In the present embodiment, the clamp member 46 holds in between the tube 18 which is connected to the vein side end 17B of the blood vessel 17 via the second plug 42.

Now, a blood perfusion method using the pseudo human body device 11 according to the present embodiment is described. The perfusion method according to the present embodiment is similar to that in the first embodiment from its start to the step of perfusing the physiological saline to the organ 12 before the perfusion of the blood. After the perfusion of the blood to the organ 12, the vein side end 17B of the blood vessel 17 or the lumen of the tube 18 is closed or narrowed by the clamp member 46 at a desired point of time. The lumen is closed by strongly grasping with the clamp member 46, and is narrowed by weakly grasping with the clamp member 46. The blood is perfused in the organ 12 in this state to evaluate the equipment. The conditions and others in the perfusion of the blood are substantially similar to those in the first embodiment.

According to the third embodiment, the pseudo human body device 11 comprises the clamp member 46 which can close the vein side end 17B of the blood vessel 17 or the lumen of the tube 18 or produce outflow resistance. According to this configuration, the vein side end 17B or the tube 18 is closed or narrowed by the operation of the clamp member 46 so that the blood flow volume inside the living tissue 26 can be temporarily increased without the operation of adjusting the supply pressure by setting the pump unit 21. Thus, when the blood pressure has decreased in the organ 12 due to some factors, the blood flow volume is quickly recovered, so that desired blood pressure can be maintained in the organ 12. According to the configuration and functions described above, it is possible to manufacture the pseudo human body device 11 which can temporarily reproduce a large volume of bleeding.

The pseudo human body device 11 comprises the circular cylindrical first plug 41 intervening between the artery side end 17A of the blood vessel 17 and the first branch 35. The first plug 41 has the first circular cylindrical portion 43 having an inside diameter smaller than the inside diameter of the first branch 35, the first minor diameter portion 44 projecting inward from the first circular cylindrical portion 43 to constitute an inside diameter smaller than the inside diameter of the first circular cylindrical portion 43, and the inclined portion 45 which is oblique to smoothly connect the first circular cylindrical portion 43 and the first minor diameter portion 44.

According to this configuration, it is possible to prevent a portion (e.g. a step) in which the inside diameter suddenly changes from being provided in the part which connects the artery side end 17A of the blood vessel 17 and the first branch 35. Thus, more blood flow can be supplied to the inside of the organ 12 by the reduction of a pressure loss that is made in the first plug 41. Consequently, it is possible to manufacture the pseudo human body device 11 which can reproduce a scale of bleeding which exceeds the supply pressure generated by the pump unit 21 in contrast to the first embodiment.

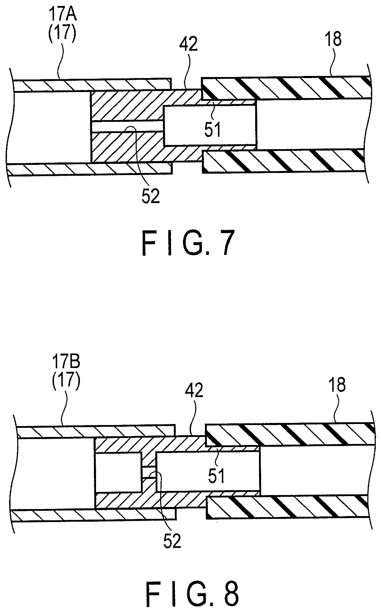

The clamp member 46 can be modified as shown in FIG. 6 and FIG. 7. In this modification (first modification), the second plug 42 having a special shape is used instead of the clamp member 46. That is, the pseudo human body device 11 comprises the second plug 42, and the second plug 42 is attached to the vein side end 17B of the blood vessel 17.

The second plug 42 is formed, for example, into a circular cylindrical shape by a synthetic resin material. The second plug 42 has a second circular cylindrical portion 51 having an inside diameter less than or equal to the inside diameter of the tube 18, and a second minor diameter portion 52 projecting inward from the second circular cylindrical portion 51 to constitute an inside diameter smaller than the inside diameter of the second circular cylindrical portion 51. The second minor diameter portion 52 is continuously formed to account for substantially half of the length of the second plug 42 in the longitudinal direction of the second plug 42. In the first modification (FIG. 6 and FIG. 7) and a later-described second modification (FIG. 8), no tube 18 may be provided.

According to the first modification, the pseudo human body device 11 comprises the circular cylindrical second plug 42 attached to the vein side end 17B of the blood vessel 17. The second plug 42 has the second circular cylindrical portion 51 having an inside diameter smaller than the inside diameter of the second branch 36, and the second minor diameter portion 52 projecting inward from the second circular cylindrical portion 51 to constitute an inside diameter smaller than the inside diameter of the second circular cylindrical portion 51.

According to this configuration, a structure which serves as a resistance during the flow of the blood in the second plug 42 can be provided in the second plug 42, which makes it difficult for the blood flowing in the second plug 42 to flow. Thus, it is possible to manufacture the pseudo human body device 11 in which the outflow resistance is provided for the blood flowing out of the second plug 42 and which can thereby ensure a greater blood flow volume in the organ 12 and reproduce massive bleeding as in the case where the vein side end 17B or the tube 18 is weakly grasped by the clamp member 46.

The structure of the second plug 42 can be modified as shown in FIG. 8. In this modification (second modification), the second minor diameter portion 52 is formed into a thin wall shape at an intermediate position in the longitudinal direction of the second plug 42. When the second minor diameter portion 52 is configured as in the second modification, advantageous effects similar to those in the first modification described above can be obtained.

### [Fourth Embodiment]

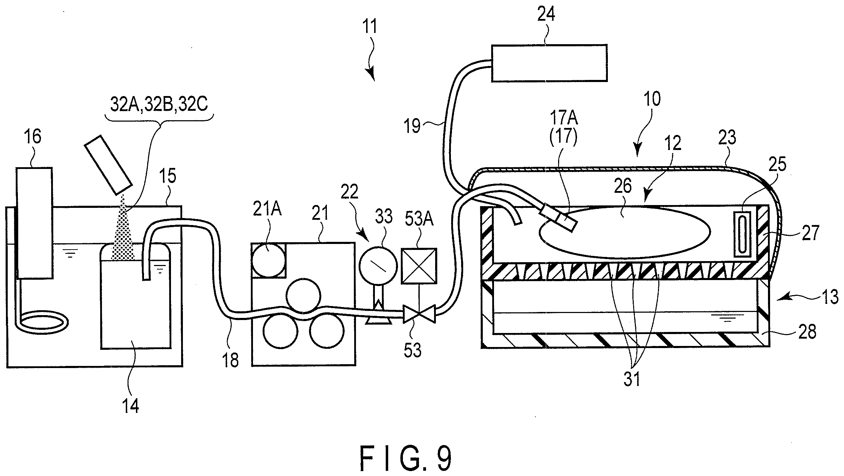

The pseudo human body device 11 according to the fourth embodiment is described with reference to FIG. 9. The pseudo human body device 11 according to the fourth embodiment is different from that according to the first embodiment in that an electromagnetic valve 53 is provided along the tube 18. However, the pseudo human body device 11 according to the fourth embodiment is the same as that according to the first embodiment in other respects. Therefore, the differences between the first embodiment and the fourth embodiment are mainly described, and the same parts are neither shown in the drawings nor described.

The electromagnetic valve 53 has, for example, a control unit 53A with a timer function. This control unit 53A opens and closes a flow path in the electromagnetic valve 53, for example, at predetermined intervals. In the present embodiment, the control unit 53A opens and closes the electromagnetic valve 53, for example, to repeat the process of opening the flow path for one second and the process of closing the flow path for the subsequent one second. The electromagnetic valve 53 is an example of a blood flow adjusting unit which can switch on and off the flow of the blood in the tube 18 at predetermined intervals.

Now, a manufacturing method of the pseudo human body device 11 and a blood perfusion method using this pseudo human body device 11 according to the present embodiment are described. The manufacturing method of the pseudo human body device 11 according to the present embodiment is the same as the method of the first embodiment from its start to the step of perfusing the physiological saline to the organ 12 before the perfusion of the blood. The blood is then perfused in the organ 12, for example, for 5 to 15 minutes. The rotation number of the pump unit 21 is 6 rpm. The electromagnetic valve 53 is repeatedly opened and closed at intervals of one second. Thus, the blood flow can be pulsated. Therefore, even if a thrombus is produced in the blood vessel 17, the thrombus can be removed by the pulsation. The total perfusion volume of the blood is smaller than that in the first embodiment because of the intermittent blood flow, and is, for example, 50 ml to 500 ml.

According to the fourth embodiment, the pseudo human body device 11 comprises the blood flow adjusting unit which is provided along the tube 18 and which can switch on and off the flow of the blood in the tube 18 at predetermined intervals. According to this configuration, the blood flow can be pulsated, and even when the blood vessel is, for example, clogged with a thrombus, the thrombus can be removed. Thus, there is a smaller influence of the precipitation of protein in the thrombus or the blood and impurities mixed in the blood, so that it is possible to inhibit the variation in the easiness of the flow of blood during the evaluation of the equipment. It is therefore possible to reproduce a more stable bleeding phenomenon. It is possible to improve the blood flow and reproduce massive bleeding even under a relatively low supply pressure of blood. If the blood flow is actively pulsated by the blood flow adjusting unit at predetermined intervals, the blood pulsation of a human body can also be significantly reproduced. This brings about another advantage, it is possible to reproduce a bleeding phenomenon involving the pulsation generated in an actual human body, and evaluate the equipment in a situation closer to an actual scene.

### [Fifth Embodiment]

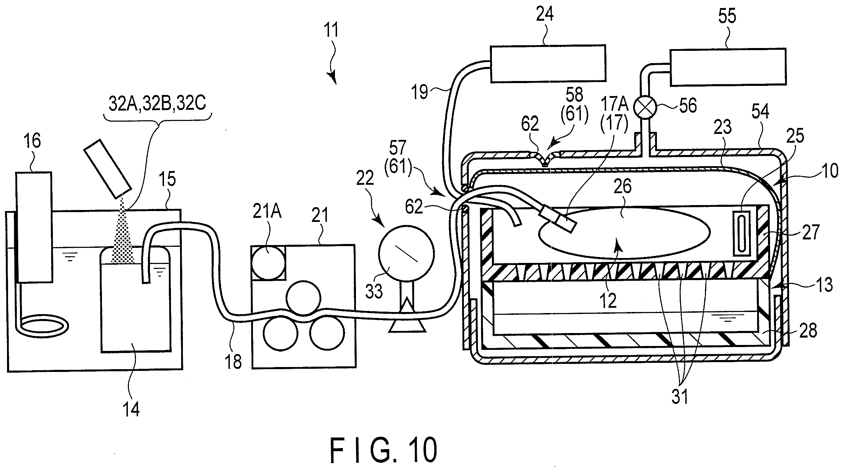

The pseudo human body device 11 according to the fifth embodiment is described with reference to FIG. 10. The pseudo human body device 11 according to the fifth embodiment is different from that according to the first embodiment in having a chamber 54 which surrounds the mounting unit 13, and a second pump unit 55 which lets the air out of the chamber 54. However, the pseudo human body device 11 according to the fifth embodiment is the same as that according to the first embodiment in other respects. Therefore, the differences between the first embodiment and the fifth embodiment are mainly described, and the same parts are neither shown in the drawings nor described.

In addition to the configuration according to the first embodiment, the pseudo human body device 11 further comprises the chamber 54 which surrounds the mounting unit 13 and the organ 12 held in the mounting unit 13, the second pump unit 55 which lets the air out of the chamber 54, and a valve 56 provided along a path which connects the chamber 54 and the second pump unit 55.

The chamber 54 is formed into a box shape by a metallic material. The chamber 54 has a first port 57 through which the tube 18 and the second tube 19 are passed, and a second port 58 through which the treatment device is passed. The first port 57 and the second port 58 respectively constitute connection openings 61 which connect the inside of the chamber 54 to the outside. The first port 57 and the second port 58 respectively have flexible members 62 which show rubber-like elasticity, and can airtightly divide the inside of the chamber 54 from the outside by the flexible members 62.

The second pump unit 55 comprises what is known as a vacuum pump, and deaerates the chamber 54 so that the air pressure inside the chamber 54 can be lower than that of the outside.

Now, a manufacturing method of the pseudo human body device 11 and a blood perfusion method using this pseudo human body device 11 according to the present embodiment are described. The manufacturing method of the pseudo human body device 11 according to the present embodiment is the same as the method of the first embodiment. The blood is perfused in the organ 12 under substantially the same conditions as those in the first embodiment. In the present embodiment, the second pump unit 55 is actuated to decompress the organ 12 during the perfusion of the blood. The blood vessel 17 is thereby dilated so that the blood easily flows through the blood vessel 17.

According to the present embodiment, the pseudo human body device 11 comprises the chamber 54 which surrounds the organ 12 to decompress the organ 12 and which has the ports to pass the treatment device through the chamber 54. According to this configuration, the organ 12 can be decompressed by the chamber 54, and the blood vessel 17 in the organ 12 can be dilated to improve the blood flow in the blood vessel 17. Thus, there is a smaller influence of the thrombus, the precipitation of protein in the blood, and impurities mixed in the blood, so that it is possible to inhibit the variation in the easiness of the flow of blood during the evaluation of the equipment. It is therefore possible to reproduce a more stable bleeding phenomenon. It is possible to provide the pseudo human body device 11 which can reproduce massive bleeding.

Although the second pump unit 55 comprises the vacuum pump in the present embodiment, the second pump unit 55 may comprise a pressurization pump. In the case of this modification, the second pump unit 55 pressurizes the chamber 54 so that a stimulus which shrinks the blood vessel of the organ 12 can be applied. It is therefore possible to provide the pseudo human body device 11 which can improve the blood flow in the blood vessel 17 and reproduce a more stable bleeding phenomenon and which can reproduce massive bleeding.

### [Sixth Embodiment]

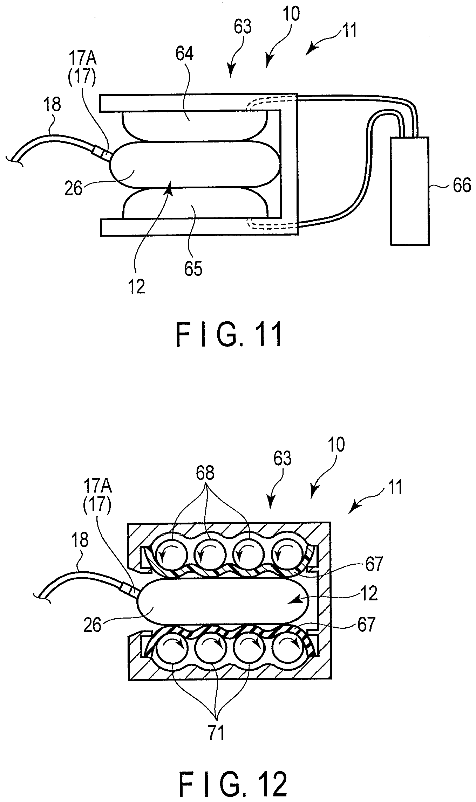

The pseudo human body device 11 according to the sixth embodiment is described with reference to FIG. 11. The pseudo human body device 11 according to the sixth embodiment is different from that according to the first embodiment in having stimulus supply means 63 for applying a physical stimulus from the outside of the organ 12. However, the pseudo human body device 11 according to the sixth embodiment is the same as that according to the first embodiment in other respects. Therefore, the differences between the first embodiment and the sixth embodiment are mainly described, and the same parts are neither shown in the drawings nor described.

The stimulus supply means 63 is disposed on the first tray 27 of the mounting unit 13. In FIG. 11, components other than the stimulus supply means 63, the organ 12, and the first tube 18 are omitted. The stimulus supply means 63 has a first balloon 64 which contacts one surface of the organ 12, a second balloon 65 which contacts the other surface of the organ 12 opposite to the one surface, and a pressurization pump 66 which supplies air to the first balloon 64 and the second balloon 65.

Now, a manufacturing method of the pseudo human body device 11 and a blood perfusion method using this pseudo human body device 11 according to the present embodiment are described. The manufacturing method of the pseudo human body device 11 according to the present embodiment is the same as that in the first embodiment. The blood is perfused in the organ 12 under substantially the same conditions as those in the first embodiment. In the present embodiment, during the perfusion of the blood, the first balloon 64 and the second balloon 65 of the stimulus supply means 63 are inflated to apply a physical stimulus from the outside of the organ 12 and thereby improve the blood flow.

According to the present embodiment, the pseudo human body device 11 comprises the stimulus supply means 63 for applying a physical stimulus from the outside of the organ 12. According to this configuration, a thrombus (clog) in the organ 12 can be eliminated so that the blood easily flows through the blood vessel 17.

The stimulus supply means 63 according to the present embodiment can be modified as shown in FIG. 12. In this modification, the stimulus supply means 63 has a first roller unit 68 which rotates counterclockwise to draw the organ 12 via a flexible membranous member 67, and a second roller unit 71 which rotates clockwise to draw the organ 12 via the flexible membranous member 67, as shown in FIG. 12. According to this modification as well, a physical stimulus is applied to the organ 12 and the blood vessel 17 from the outside, and a thrombus (clog) in the blood vessel 17 can be eliminated so that the blood easily flows through the blood vessel 17.

### [Seventh Embodiment]

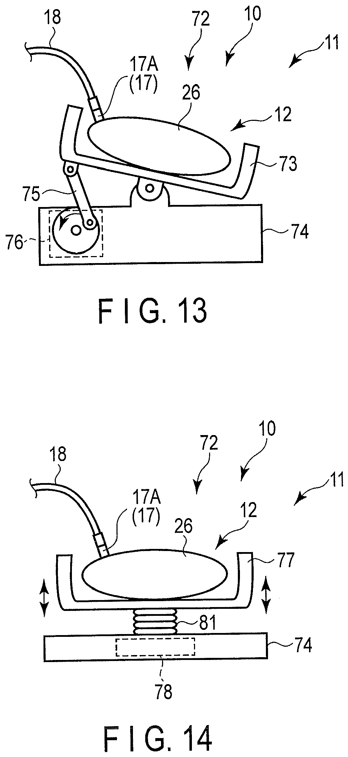

The pseudo human body device 11 according to the seventh embodiment is described with reference to FIG. 13. The pseudo human body device 11 according to the seventh embodiment is different from that according to the first embodiment in having a vibration applying unit 72 which applies vibration to the organ 12. However, the pseudo human body device 11 according to the sixth embodiment is the same as that according to the first embodiment in other respects. Therefore, the differences between the first embodiment and the seventh embodiment are mainly described, and the same parts are neither shown in the drawings nor described.

The vibration applying unit 72 is disposed on the first tray 27 of the mounting unit 13. In FIG. 13, components other than the vibration applying unit 72, the organ 12, and the first tube 18 are omitted. The vibration applying unit 72 has a shake plate 73 which supports the organ 12, a support table 74 which supports the shake plate 73 so that the shake plate 73 can be inclined, a link mechanism 75 connected to the shake plate 73, and a motor 76 which applies torque to the link mechanism 75.

A manufacturing method of the pseudo human body device 11 and a blood perfusion method using this pseudo human body device 11 according to the present embodiment are described. The manufacturing method of the pseudo human body device 11 according to the present embodiment is the same as that in the first embodiment. The blood is perfused in the organ 12 under substantially the same conditions as those in the first embodiment. In the present embodiment, during the perfusion of the blood, the motor 76 and the link mechanism 75 of the vibration applying unit 72 rotate to apply vibration to the organ 12 and thus improve the blood flow in the blood vessel 17.

According to the present embodiment, the pseudo human body device 11 comprises the vibration applying unit 72 which applies vibration to the organ 12. According to this configuration, a thrombus (clog) in the blood vessel 17 can be eliminated by the vibration applied to the organ 12 so that the blood easily flows through the blood vessel 17.

The vibration applying unit 72 according to the present embodiment can be modified as shown in FIG. 14. In this modification, the vibration applying unit 72 has a vibrating plate 77 which supports the organ 12, the support table 74 which has a vibrator 78 therein, and a spring-shaped member 81 intervening between the vibrating plate 77 and the vibrator 78. In the present embodiment, during the perfusion of the blood, the vibrator 78 is actuated to apply vibration to the organ 12 and thus improve the blood flow in the blood vessel 17.

### [Eighth Embodiment]

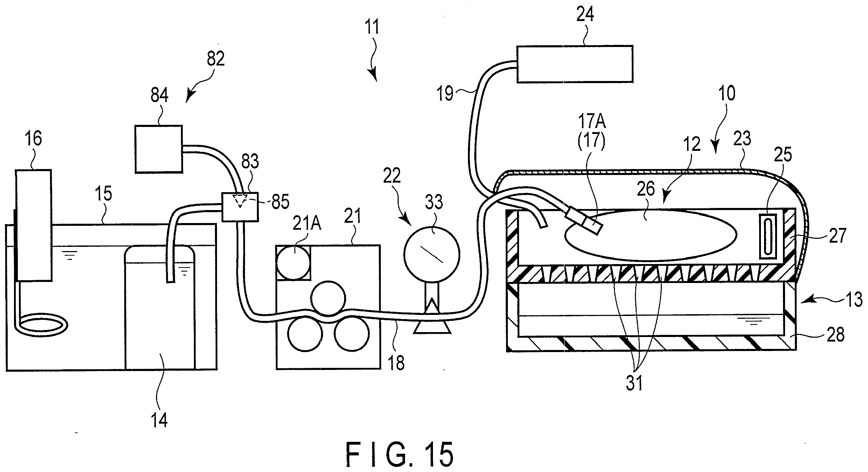

The pseudo human body device according to the eighth embodiment is described with reference to FIG. 15. The pseudo human body device 11 according to the eighth embodiment is different from that according to the first embodiment in having a microbubble generating device 82 which mixes microbubbles into blood. However, the pseudo human body device 11 according to the sixth embodiment is the same as that according to the first embodiment in other respects. Therefore, the differences between the first embodiment and the seventh embodiment are mainly described, and the same parts are neither shown in the drawings nor described.

The pseudo human body device 11 has the microbubble generating device 82 (microbubble mixing unit) intervening at a position along the tube 18. The tube 18 is formed into a tubular shape by, for example, a transparent resin so that the flow of the blood running therethrough can be visually recognized. The tube 18 has a box-shaped mixing unit 83 along this tube 18, and is connected to the microbubble generating device 82 via the mixing unit 83.

The microbubble generating device 82 includes a device main unit 84 which sends air, and a nozzle 85 provided in the mixing unit 83. The nozzle 85 has pores for generating microbubbles in the mixing unit 83, and is formed by, for example, a sintered body. In the present embodiment, a microbubble refers to, for example, an air bubble having a diameter of 0.01 mm to 0.1 mm. The configuration of the microbubble generating device 82 is one example, and a device which generates microbubbles by some other method is also possible.

A manufacturing method of the pseudo human body device 11 and a blood perfusion method using this pseudo human body device 11 according to the present embodiment are described. The manufacturing method of the pseudo human body device 11 according to the present embodiment is the same as that in the first embodiment. The blood is perfused in the organ 12 under substantially the same conditions as those in the first embodiment. In this instance, the microbubble generating device 82 is actuated to add microbubbles to the blood and thus improve the blood flow in the blood vessel.

According to the present embodiment, the pseudo human body device 11 comprises the microbubble mixing unit which intervenes in the tube 18 and which mixes microbubbles into the blood. According to this configuration, the easiness of the flow of blood running through the blood vessel 17 is improved by the microbubbles, and the blood flow can be improved even under low perfusion pressure. As a result, it is possible to reproduce massive bleeding from the living tissue 26.

### [Ninth Embodiment]

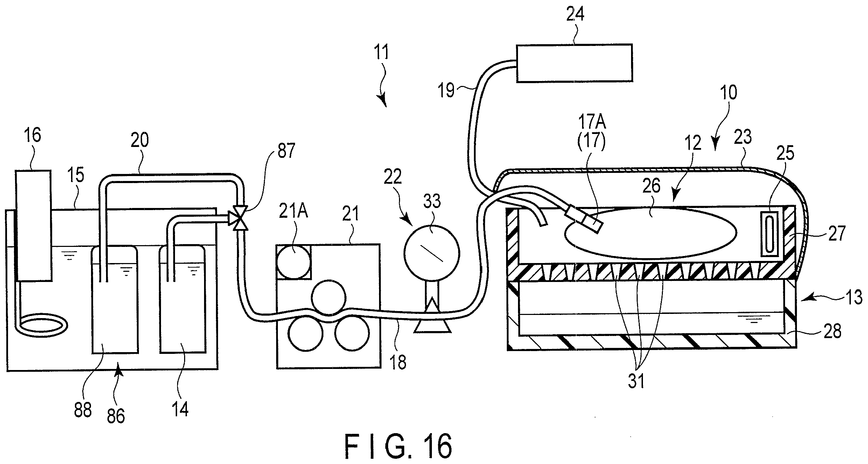

The pseudo human body device 11 according to the ninth embodiment is described with reference to FIG. 16. The pseudo human body device 11 according to the ninth embodiment is different from that according to the first embodiment in having a physiological saline mixing unit which mixes a physiological saline into blood. However, the pseudo human body device 11 according to the ninth embodiment is the same as that according to the first embodiment in other respects. Therefore, the differences between the first embodiment and the ninth embodiment are mainly described, and the same parts are neither shown in the drawings nor described.

The pseudo human body device 11 has a physiological saline mixing unit 86 intervening in the tube 18. The tube 18 is formed into a tubular shape by, for example, a transparent resin so that the flow of the blood running therethrough can be visually recognized. The tube 18 has a mixing valve (three-way valve) along this tube 18, and is connected to the physiological saline mixing unit 86 via a mixing valve 87. The mixing valve 87 can mix blood and a physiological saline at a desired ratio. The mixing ratio between the blood and the physiological saline can be suitably adjusted by operating an operation unit provided in the mixing valve 87. In the present embodiment, blood in which the physiological saline is mixed at, for example, 10 to 20 volume percent concentration is used.

The physiological saline mixing unit 86 has a physiological saline bottle 88 which retains a physiological saline therein, and a third tube 20 which is connected to the mixing valve 87.

A manufacturing method of the pseudo human body device 11 and a blood perfusion method using this pseudo human body device according to the present embodiment are described. The manufacturing method of the pseudo human body device 11 according to the present embodiment is similar to that in the first embodiment. The blood is perfused in the organ 12 under substantially the same conditions as those in the first embodiment. In this instance, the physiological saline is mixed in the blood used, so that the viscosity of the blood is reduced to improve the blood flow in the blood vessel 17.

According to the present embodiment, the pseudo human body device 11 comprises the physiological saline mixing unit 86 which intervenes in the tube 18 and which mixes the physiological saline into the blood. According to this configuration, the viscosity of the blood can be reduced as needed, and the blood flow can be improved even under low perfusion pressure. As a result, it is possible to reproduce massive bleeding from the living tissue 26.

The present invention is not limited to the embodiments described above, and modifications can be suitably made without departing from the spirit thereof. Moreover, it should be appreciated that the pseudo human body devices according to the embodiments can be combined to constitute one pseudo human body device.

Another pseudo human body device which achieves the present invention is additionally set forth below.

### [1]

A pseudo human body device comprising:
an organ of a domestic animal having a living tissue and a blood vessel extending from the living tissue;
a blood collecting unit which collects blood to be sent to the blood vessel;
a tube which connects the blood vessel to the blood collecting unit;
a pump unit which sends the blood to the blood vessel from the blood collecting unit; and
a heater/humidifier unit which sends steam of a temperature higher than a room temperature to the organ to humidify the organ and bring the organ to a temperature of 20°C to 50°C.

### Reference Signs List

11: pseudo human body device, 12: organ, 14: blood bottle, 15: constant temperature bath, 16: heater, 17: blood vessel, 18: tube, 21: pump unit, 22: pressure sensor, 23: cover, 24: high-temperature steam generator, 26: living tissue, 32A: anticoagulant, 32B: thrombolytic agent, 32C: vasodilator, 34 body, 35: first branch, 36 second branch, 37: switching valve, 41: first plug, 42: second plug, 43: first circular cylindrical portion, 44: first minor diameter portion, 45: inclined portion, 46: clamp member, 51: second circular cylindrical portion, 52: second minor diameter portion, 53: electromagnetic valve, 54: chamber, 57: first port, 58: second port, 63: stimulus supply means, 72: vibration applying unit, 82: microbubble generating device, 86: physiological saline mixing unit

## Claims

1. A pseudo human body device comprising:
an animal-derived organ having a living tissue and a blood vessel extending from the living tissue;
a blood collecting unit which collects blood to be sent to the blood vessel;
a tube which connects the blood vessel to the blood collecting unit;
a pump unit which is provided along the tube and which sends the blood to the blood vessel from the blood collecting unit; and
a housing unit which houses the organ and which sends steam of a temperature higher than a room temperature to the organ to humidify the organ and bring the organ to a temperature of 20°C to 50°C.

2. The pseudo human body device according to claim 1, wherein the tube comprises
a part connected to the blood collecting unit,
a tubular first branch extending from the part and connected to an artery side end of the blood vessel,
a tubular second branch extending from the part and connected to a vein side end of the blood vessel, and
a switching valve which intervenes between the part, the first branch, and the second branch and which is configured to circulate the blood to one of the first branch and the second branch.

3. The pseudo human body device according to claim 2, further comprising a sensor unit which is provided along the tube and which senses the pressure of the blood,
wherein the switching valve circulates the blood to the first branch in a normal state, and circulates the blood to the second branch when the pressure of the blood sensed by the sensor unit is more than predetermined pressure which is higher than the pressure of the blood in the normal state.

4. The pseudo human body device according to claim 1, further comprising a temperature adjusting unit which applies heat to the blood collecting unit to warm the blood to a predetermined temperature and keep the blood warm.

5. The pseudo human body device according to claim 1, wherein at least one of an anticoagulant, a thrombolytic agent, a vasodilator is mixed in the blood.

6. The pseudo human body device according to claim 1, further comprising a clamp member configured to close or narrow the vein side end of the blood vessel.

7. The pseudo human body device according to claim 1, further comprising a circular cylindrical first plug intervening between the artery side end of the blood vessel and the tube,
wherein the first plug comprises
a first circular cylindrical portion having an inside diameter less than or equal to the inside diameter of the tube,
a first minor diameter portion projecting inward from the first circular cylindrical portion to constitute an inside diameter smaller than the inside diameter of the first circular cylindrical portion, and
an inclined portion which is oblique to smoothly connect the first circular cylindrical portion and the first minor diameter portion.

8. The pseudo human body device according to claim 1, further comprising a circular cylindrical second plug attached to the vein side end of the blood vessel,
wherein the second plug comprises
a second circular cylindrical portion having an inside diameter less than or equal to the inside diameter of the tube, and
a second minor diameter portion projecting inward from the second circular cylindrical portion to constitute an inside diameter smaller than the inside diameter of the second circular cylindrical portion.

9. The pseudo human body device according to claim 1, further comprising a blood flow adjusting unit which is provided along the tube and which is configured to switch on and off the flow of the blood in the tube at predetermined intervals.

10. The pseudo human body device according to claim 1, further comprising a chamber which surrounds the organ to decompress the organ and which has ports to pass a treatment device through the chamber.

11. The pseudo human body device according to claim 1, further comprising stimulus supply means for applying a physical stimulus to the organ from the outside.

12. The pseudo human body device according to claim 1, further comprising a vibration applying unit which applies vibration to the organ.

13. The pseudo human body device according to claim 1, further comprising a microbubble mixing unit which intervenes in the tube and which mixes microbubbles into the blood.

14. The pseudo human body device according to claim 1, further comprising a physiological saline mixing unit which intervenes in the tube and which mixes a physiological saline into the blood.
